# DEMANDE DE BREVET EUROPEEN

(11) **EP 3 000 394 A1**
(43) Date de publication de la demande: **30.03.2016**
(21) Numéro de dépôt: 15186062.4
(22) Date de dépôt: 21.09.2015
(51) Int. Cl.: A61B 5/103, G06K 7/14, G01J 3/52

(54) **DISPOSITIF ET METHODE DE CALIBRAGE COLORIMETRIQUE D'UNE IMAGE**

(30) Priorité: 23.09.2014 FR 1458931
(71) Demandeur: Pixience, 31000 Toulouse (FR)
(72) Inventeur: DZIK, Nicolas, 31650 SAINT ORENS DE GAMEVILLE (FR)
(74) Mandataire: Argyma

(57) **Abrégé**

Un dispositif de calibrage colorimétrique (1) comportant un ensemble (2) de zones colorimétriques (20) de couleurs prédéterminées et au moins une première cible de localisation (C1, C2) située au voisinage dudit ensemble (2) de zones colorimétriques (20).

## Description

### DOMAINE TECHNIQUE GENERAL ET ART ANTERIEUR

La présente invention concerne le domaine du calibrage colorimétrique d'une image, en particulier, dans le but d'obtenir une image pouvant être utilisée pour suivre une pathologie dermatologique.

Les couleurs d'une image peuvent varier du fait des variations des conditions d'éclairage et/ou du réglage de la caméra d'acquisition de laquelle l'image est issue. Aussi, il est nécessaire de calibrer les couleurs d'une image afin de pouvoir la comparer de manière pertinente à une image étalon. Une telle comparaison possède une fiabilité élevée et permet de faciliter l'établissement d'un diagnostic, en particulier, par un médecin dermatologue.

En pratique, pour obtenir une image calibrée de la peau d'un patient, un dermatologue peut utiliser une caméra d'acquisition perfectionnée qui comporte des moyens internes de calibration. Une telle caméra d'acquisition perfectionnée présente un coût important et n'est pas à disposition de chaque médecin dermatologue. Par ailleurs, pour des applications en télémédecine, il n'est pas envisageable de requérir que chaque patient s'équipe d'une telle caméra d'acquisition perfectionnée.

Aussi, une alternative consiste à prendre avec une caméra classique, par exemple une caméra d'ordinateur, une photographie de la peau du patient dans laquelle est disposée une mire colorimétrique. Une mire colorimétrique, connue par exemple sous la dénomination commerciale XRITE, comporte un ensemble de zones colorimétriques dont les couleurs sont parfaitement déterminées afin de servir d'étalon colorimétrique. En pratique, les zones colorimétriques de la mire sont organisées sous la forme d'un tableau (lignes et colonnes) et la position d'une zone colorimétrique est déterminée de manière précise dans ledit tableau (coordonnées ligne et colonne).

Pour calibrer les couleurs d'une photographie, une méthode de calibrage est traditionnellement mise en oeuvre par un ordinateur. La méthode comporte une étape de détection de la position de chaque zone colorimétrique de la mire.

Une fois la position de chaque zone colorimétrique détectée, la méthode de calibrage comporte une étape de lecture des couleurs des zones colorimétriques et une étape de comparaison des couleurs lues avec les couleurs étalon afin de déterminer un écart colorimétrique. Ensuite, le procédé comporte une étape de traitement de la photographie qui est fonction de l'écart colorimétrique mesuré afin d'obtenir une image calibrée. Une telle méthode de calibrage est connue de l'homme du métier, en particulier, par la demande de brevet US 8073248.

Cette méthode de calibrage possède néanmoins de nombreux inconvénients. En particulier, l'étape de détection de la position de chaque zone colorimétrique de la mire est difficile à mettre en oeuvre et requiert une puissance de calcul importante. En pratique, chaque zone colorimétrique doit être détectée de manière individuelle, ce qui nécessite un temps de calcul important et empêche tout traitement en temps réel.

En outre, la détection des zones colorimétriques de la mire n'est pas robuste et dépend des couleurs présentes dans la photographie à calibrer. De plus, la mire colorimétrique n'est pas toujours détectable lorsqu'elle est placée en biais par rapport à la caméra, ce qui présente un autre inconvénient.

Par ailleurs, une mire colorimétrique ne permet pas de mesurer de manière fiable la géométrie des objets visibles sur la photographie. Aussi, il est nécessaire de faire apparaître un outil de mesure supplémentaire sur la photographie (en plus de la mire colorimétrique) afin de permettre de réaliser des mesures géométriques de pathologies d'un patient.

L'invention a donc pour but de remédier à ces inconvénients en proposant un dispositif et un procédé de calibrage afin d'améliorer le diagnostic d'une pathologie, en particulier, pour des applications de télémédecine.

### PRESENTATION GENERALE DE L'INVENTION

A cet effet, l'invention concerne un dispositif de calibrage colorimétrique comportant un ensemble de zones colorimétriques de couleurs prédéterminées.

L'invention est remarquable en ce qu'il comporte au moins une première cible de localisation située au voisinage dudit ensemble de zones colorimétriques.

Une telle localisation indirecte des zones colorimétriques est plus rapide qu'une localisation directe selon l'art antérieur. Grâce à la cible de localisation, il est possible de localiser de manière robuste et pratique les zones colorimétriques de couleurs prédéterminées. Cela est particulièrement avantageux pour réaliser un calibrage colorimétrique dans un flux vidéo en temps réel. Il n'est avantageusement plus nécessaire de localiser individuellement chaque zone colorimétrique dans l'image comme dans l'art antérieur.

De préférence, chaque cible de localisation comporte au moins un motif orienté afin de définir un référentiel précis pour faciliter la lecture des couleurs des zones colorimétriques.

De manière préférée, le dispositif de calibrage comporte au moins une première cible de localisation et une deuxième cible de localisation afin de former un référentiel robuste pour la lecture des couleurs des zones colorimétriques. De préférence encore, la première cible de localisation et la deuxième cible de localisation sont situées à des extrémités opposées dudit dispositif de calibrage afin d'améliorer la précision du référentiel.

De manière préférée, ledit ensemble de zones colorimétriques est situé entre ladite première cible de localisation et ladite deuxième cible de localisation afin de faciliter la rectification géométrique des zones colorimétriques grâce au référentiel des cibles de localisation. Le référentiel obtenu est ainsi particulièrement pertinent au regard de l'ensemble des zones colorimétriques. Avantageusement, l'ensemble de zones colorimétriques, la première cible de localisation et la deuxième cible de localisation appartiennent à un même plan.

De manière préférée, la première cible de localisation et la deuxième cible de localisation sont différentes pour former un référentiel orienté.

Selon un aspect de l'invention, le dispositif comporte au moins trois cibles de localisation non alignées définissant un contour à l'intérieur duquel ledit ensemble de zones colorimétriques est situé.

De préférence, chaque dispositif de calibrage est stérile afin de pouvoir être approché au plus près d'une zone infectée.

Selon un autre aspect de l'invention, chaque cible de localisation est noire et blanche afin de posséder un fort contraste et ainsi faciliter sa localisation et sa lecture.

De manière préférée, chaque cible de localisation se présente sous la forme d'une cible intelligente du type QR Code, code-barres ou datamatrix.

Selon un aspect préféré de l'invention, le dispositif de calibrage comporte un support, sur lequel est située au moins une cible de localisation comportant un logement, et une cartouche, montée de manière amovible dans le logement dudit support sur laquelle est situé l'ensemble de zones colorimétriques. Ainsi, une mire colorimétrique selon l'art antérieur peut être utilisée en tant que cartouche.

L'invention concerne également une méthode de calibrage colorimétrique d'une image brute dans laquelle est positionné un dispositif de calibrage tel que présenté précédemment, la méthode comportant :
- une étape de localisation d'au moins une cible de localisation dudit dispositif de calibrage dans ladite image brute afin de définir un référentiel brut ;
- une étape de rectification géométrique de ladite image brute à partir dudit référentiel brut afin d'obtenir une image rectifiée ;
- une étape de lecture des couleurs desdites zones colorimétriques dans ladite image rectifiée afin de déterminer un écart colorimétrique entre les couleurs lues et lesdites couleurs prédéterminées desdites zones colorimétriques ; et
- une étape de calibrage colorimétrique de ladite image brute à partir dudit écart colorimétrique déterminé afin d'obtenir une image calibrée.

Grâce aux étapes de localisation de la cible et de rectification géométrique, l'étape de lecture des couleurs desdites zones colorimétriques est simple à mettre en oeuvre, ce qui accélère de manière importante l'ensemble de la méthode de calibrage et permet une application en temps réel.

De préférence, la méthode comporte une étape de détermination d'une matrice de transformation géométrique apte à transformer la cible de localisation de l'image brute en une cible de localisation standardisée, l'image brute est rectifiée grâce à la matrice de transformation géométrique. De préférence encore, la méthode comporte une étape de détermination d'une matrice de transformation géométrique pour un ensemble de cibles de localisation de l'image brute. Ainsi, l'image brute subit une rectification robuste et précise étant donné que la surface des cibles est plus importante, ce qui facilite une détermination précise de la matrice de transformation géométrique.

L'invention vise aussi un procédé de calibrage colorimétrique d'un flux vidéo en temps réel par une caméra dans lequel le procédé comporte une méthode de calibrage colorimétrique, telle que présentée précédemment, d'une pluralité d'images dudit flux vidéo, de préférence, de chaque image dudit flux vidéo. Ainsi, un dermatologue peut forger son diagnostic en temps réel par analyse d'un flux vidéo dans lequel une pathologie est visible selon plusieurs angles et plusieurs éclairages.

L'invention vise également un logiciel informatique mettant en oeuvre le procédé et/ ou la méthode de calibrage ainsi qu'un support informatique sur lequel est stocké ledit logiciel informatique, par exemple, un serveur informatique, un ordinateur ou un téléphone intelligent.

### PRESENTATION DES FIGURES

L'invention sera mieux comprise à la lecture de la description qui va suivre, donnée uniquement à titre d'exemple, et se référant aux dessins annexés sur lesquels :
- la figure 1 est une représentation schématique d'une première forme de réalisation d'un dispositif de calibrage selon l'invention ;
- la figure 2 est une représentation schématique d'une deuxième forme de réalisation d'un dispositif de calibrage selon l'invention ;
- la figure 3 est une représentation schématique d'une troisième forme de réalisation d'un dispositif de calibrage selon l'invention ;
- la figure 4 est une figure schématique d'un patient tenant le dispositif de calibrage de la figure 1 ;
- la figure 5 est un diagramme schématique d'une méthode de calibrage d'une image selon l'invention ; et
- la figure 6 est une représentation schématique de la rectification géométrique d'une cible de localisation.

Il faut noter que les figures exposent l'invention de manière détaillée pour mettre en oeuvre l'invention, lesdites figures pouvant bien entendu servir à mieux définir l'invention le cas échéant.

### DESCRIPTION D'UN OU PLUSIEURS MODES DE REALISATION ET DE MISE EN OEUVRE

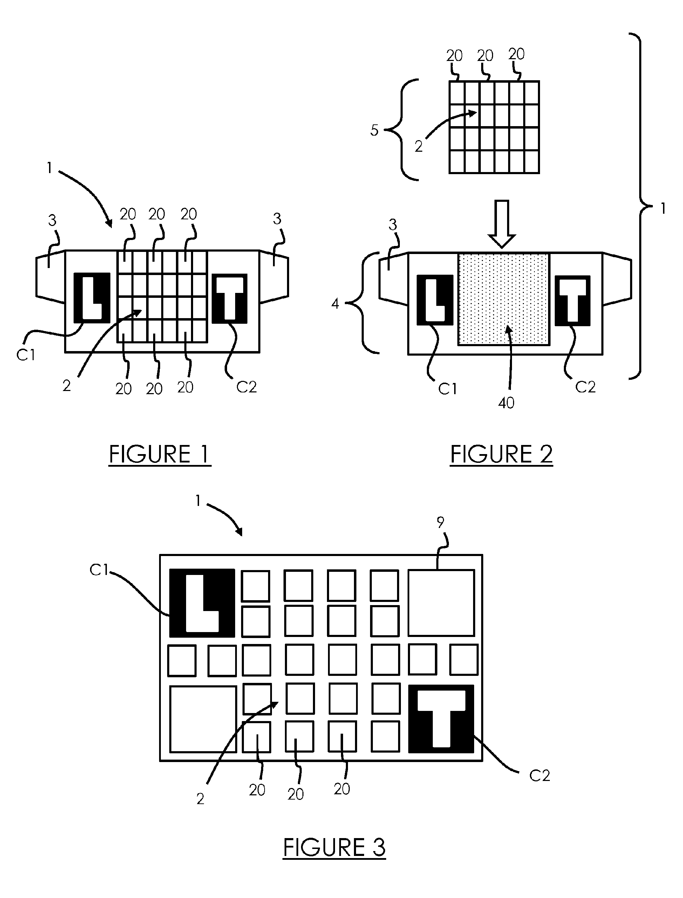

En référence à la figure 1, il est représenté de manière schématique une première forme de réalisation d'un dispositif de calibrage colorimétrique 1 selon l'invention afin de permettre un calibrage rapide et robuste des couleurs d'une image obtenue au moyen d'un appareil photographique ou d'une caméra vidéo.

Toujours en référence à la figure 1, le dispositif de calibrage colorimétrique 1 comporte un ensemble 2 de zones colorimétriques 20 de couleurs prédéterminées. Dans cet exemple, le dispositif comporte 24 zones colorimétriques 20 mais il va de soi que leur nombre pourrait être différent. Comme présenté précédemment, un tel ensemble 2 de zones colorimétriques 20 forme un étalon colorimétrique à la manière d'une mire de calibrage selon l'art antérieur. De manière préférée, chaque zone colorimétrique 20 possède une forme rectangulaire.

Dans cet exemple, le dispositif de calibrage colorimétrique 1 comporte une première cible de localisation C1 et une deuxième cible de localisation C2. Néanmoins, il va de soi qu'une unique cible de localisation ou plus de deux cibles de localisation pourraient convenir. Plus le nombre de cibles de localisation est important et plus la localisation est robuste. A l'inverse, plus le nombre de cibles est restreint et plus le dispositif de calibrage colorimétrique 1 possède un encombrement réduit, ce qui est avantageux. De manière préférée, le dispositif de calibrage colorimétrique 1 comporte 2 ou 3 cibles de localisation.

De manière préférée, chaque cible de localisation C1, C2 est située au voisinage dudit ensemble 2 de zones colorimétriques 20 afin d'améliorer la détection des zones colorimétriques 20. En effet, plus la distance est faible entre une cible de localisation C1, C2 et l'ensemble 2 de zones colorimétriques 20 plus la détection est précise. Dans cet exemple, la distance entre une cible de localisation C1, C2 et l'ensemble 2 de zones colorimétriques 20 est inférieur à 2 cm.

Les cibles de localisation C1, C2 sont éloignées l'une de l'autre et sont, de préférence, situées à des extrémités opposées du dispositif de calibrage 1 afin de faciliter la mise en oeuvre de la méthode de calibrage comme cela sera présenté par la suite. De préférence, chaque cible de localisation C1, C2 est plus grande qu'une zone colorimétrique 20 afin de faciliter sa localisation.

Comme illustré à la figure 1, ledit ensemble 2 de zones colorimétriques 20 est situé entre la première cible de localisation C1 et la deuxième cible de localisation C2 afin de faciliter la détection de l'ensemble 2 des zones colorimétriques 20 suite à la localisation des cibles de localisation C1, C2. L'ensemble 2 de zones colorimétriques 20, la première cible de localisation C1 et la deuxième cible de localisation C2 appartiennent à un même plan géométrique afin de former un plan de détection.

Lorsque le dispositif de calibrage colorimétrique 1 comporte plus de deux cibles de localisation C1, C2, les cibles ne sont pas alignées ensemble mais définissent un contour à l'intérieur duquel est situé l'ensemble 2 des zones colorimétriques 20 afin de faciliter la détection de l'ensemble 2 des zones colorimétriques 20.

Ainsi, la position des cibles de localisation C1, C2 relativement à l'ensemble 2 des zones colorimétriques 20 permet d'améliorer la détection des zones colorimétriques 20 suite à la localisation des cibles de localisation C1, C2. Autrement dit, les cibles de localisation C1, C2 permettent de former un référentiel robuste pour la détection de l'ensemble 2 des zones colorimétriques 20.

Comme cela va être dorénavant présenté, les cibles de localisation C1, C2 possèdent des caractéristiques techniques avantageuses pour faciliter leur localisation par un procédé de traitement d'image.

Toujours en référence à la figure 1, chaque cible de localisation C1, C2 possède une couleur différente de celle du dispositif de calibrage 1 afin de faciliter sa détection. De préférence, la cible de localisation C1, C2 est noire et le dispositif de calibrage 1 est blanc afin de bénéficier d'un fort contraste. Il va de soi que la réciproque pourrait également convenir.

De manière avantageuse, chaque cible de localisation C1, C2 est orientée et comporte, de préférence, un motif orienté. Dans cet exemple, le motif est blanc afin de bénéficier d'un fort contraste et ainsi faciliter sa lecture par un procédé de traitement d'image. De préférence, chaque cible de localisation C1, C2 se présente sous la forme d'une cible intelligente du type QR Code, code-barres, datamatrix ou analogues. Ainsi, l'orientation d'une cible C1, C2 peut être lue par le procédé de traitement d'image afin de déterminer l'identité et la position de chaque cible de localisation C1, C2. Une telle caractéristique est avantageuse car elle permet de définir un référentiel robuste et orienté suite à la localisation des cibles C1, C2.

Dans cet exemple, en référence à la figure 1, la première cible de localisation C1 et la deuxième cible de localisation C2 sont différentes afin de permettre de les identifier de manière rapide et robuste indépendamment de l'orientation du dispositif de calibrage colorimétrique 1. De préférence, la première cible de localisation C1 possède un motif ayant forme de « L» et la deuxième cible de localisation C2 possède un motif ayant forme de « T ». Il va de soi que d'autres motifs orientés pourraient convenir.

En référence à la figure 1, le dispositif de calibrage 1 se présente sous la forme d'une feuille, en particulier cartonnée, sur laquelle sont imprimés l'ensemble 2 des zones colorimétriques 20 et les cibles de localisation C1, C2.

Par ailleurs, le dispositif de calibrage 1 comporte au moins un organe de préhension 3 apte à permettre à un utilisateur de tenir le dispositif de calibrage 1 avec deux doigts de manière confortable et sans masquer les zones colorimétriques 20 et les cibles de localisation C1, C2. Dans cet exemple, en référence à la figure 1, le dispositif de calibrage 1 comporte deux organes de préhension 3 se présentant sous la forme de languettes en saillie.

Une deuxième forme de réalisation de l'invention est décrite en référence à la figure 2. Les références utilisées pour décrire les éléments de structure ou fonction identique, équivalente ou similaire à celles des éléments de la figure 1 sont les mêmes, pour simplifier la description. D'ailleurs, l'ensemble de la description de la forme de réalisation de la figure 1 n'est pas reprise, cette description s'appliquant aux éléments de la figure 2 lorsqu'il n'y a pas d'incompatibilités. Seules les différences notables, structurelles et fonctionnelles, sont décrites.

En référence à la figure 2, le dispositif de calibrage 1 comporte un support 4, par exemple en plastique, sur lequel sont positionnées la première cible de localisation C1 et la deuxième cible de localisation C2. Le support 4 comporte un logement 40 adapté pour recevoir une cartouche 5, montée de manière amovible dans ledit logement 40, sur laquelle est positionné l'ensemble 2 des zones colorimétriques 20. De manière préférée, la cartouche 5 est une mire de calibrage colorimétrique selon l'art antérieur. Ainsi, le support 4 peut recevoir une pluralité de cartouches différentes en fonction des besoins. En outre, étant donné que les cibles de localisation C1, C2 sont présentes sur le support 4, elles permettent de définir un référentiel précis du logement 40 du support 4 et ainsi faciliter la détection des zones colorimétriques 20 de la cartouche 5 montée dans ledit logement 40.

En référence à la figure 3 représentant une troisième forme de réalisation du dispositif de calibrage 1 selon l'invention, le dispositif de calibrage 1 se présente sous la forme d'une feuille cartonnée rectangulaire sur laquelle sont imprimés l'ensemble 2 de zones colorimétriques 20 ainsi que les cibles de localisation C1, C2 afin de limiter l'encombrement. Dans cette forme de réalisation, les cibles de localisation C1, C2 sont disposées sur des coins opposés de la carte afin de faciliter l'étape de rectification géométrique comme cela sera présenté par la suite.

En pratique, lors de l'impression des zones colorimétriques 20 d'un dispositif de calibrage 1, les couleurs étalon peuvent varier d'une impression à une autre, ce qui peut diminuer la précision de la comparaison. Afin de résoudre cet inconvénient, chaque couleur étalon d'une zone colorimétrique 20 est mesurée pour chaque lot d'impression puis la couleur étalon est associée dans une base de données à un couple de données comportant la zone colorimétrique mesurée et l'identifiant du lot d'impression.

De manière préférée, en référence à la figure 3, le dispositif de calibrage 1 comporte un identifiant de lot d'impression 9, par exemple un QRcode ou une datamatrix, pouvant être lu par un procédé de traitement d'image. Chaque dispositif de calibrage 1 peut ainsi être identifié afin de déterminer de quel lot d'impression le dispositif de calibrage 1 est issu. On peut ainsi déterminer au cours du procédé de calibrage la couleur étalon d'une zone colorimétrique 20 pour chaque lot d'impression comme cela sera présenté par la suite.

De manière avantageuse, le dispositif de calibrage 1 est stérile afin de limiter le risque d'infection lors d'une utilisation à des fins médicales.

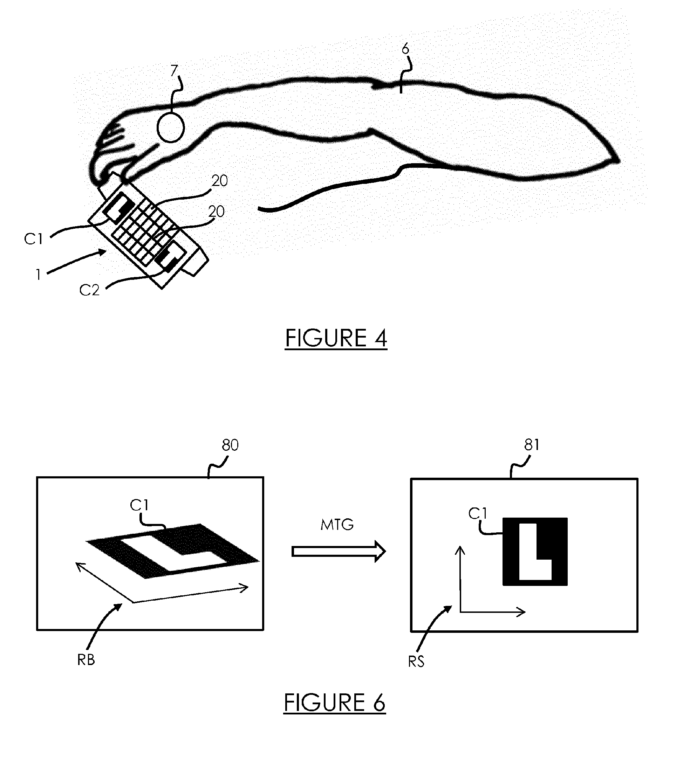

Une méthode de calibrage colorimétrique au moyen d'un dispositif de calibrage colorimétrique selon l'invention va être dorénavant présentée en référence aux figures 4 à 6.

Dans cet exemple de mise en oeuvre, en référence à la figure 4, un patient souhaite filmer une anomalie dermatologique 7 présente sur son bras 6 au moyen de la caméra vidéo de son ordinateur (non représentée). Le flux vidéo de la caméra est avantageusement transmis à un dermatologue qui peut observer l'anomalie dermatologique à distance et réaliser son diagnostic.

En pratique, comme illustré à la figure 4, le patient se positionne dans le champ de vision de la caméra vidéo afin que la scène observée par ladite caméra vidéo comporte, d'une part, l'anomalie dermatologique 7 et, d'autre part, le dispositif de calibrage colorimétrique 1. Dans cet exemple, le patient tient le dispositif de calibrage colorimétrique 1 par une languette afin que les zones colorimétriques 20 et les cibles de localisation C1, C2 soient visibles.

Dans cette mise en oeuvre, l'ordinateur du patient et l'ordinateur du dermatologue sont connectés par le réseau internet par l'intermédiaire d'un serveur d'intermédiation qui met en oeuvre une méthode de calibrage colorimétrique qui va être présentée en référence à la figure 4.

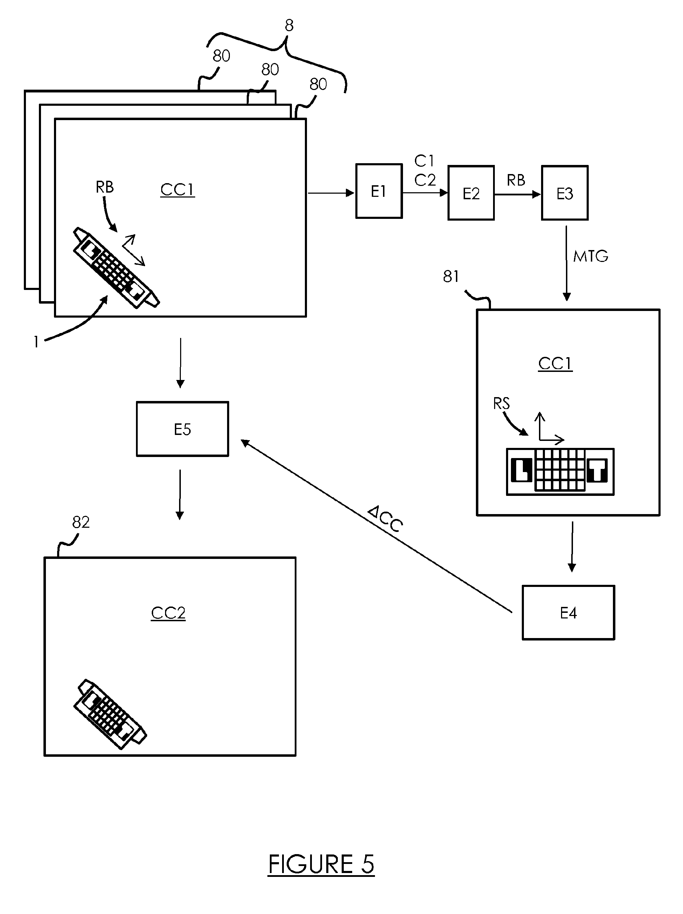

Comme illustré à la figure 5, la caméra vidéo acquiert un flux vidéo 8 comportant une pluralité d'images brutes 80 de la scène en temps réel. Chaque image brute 80 possède une configuration colorimétrique CC1 qui n'est pas calibrée.

La méthode de calibrage colorimétrique met en oeuvre une étape de localisation E1 de la première cible de localisation C1 et de la deuxième cible de localisation C2 dans une image brute 80.

La localisation des cibles C1, C2 est simple à mettre en oeuvre car elles possèdent un contraste important par rapport à la couleur du dispositif de calibrage 1. En outre, chaque cible de localisation C1, C2 possède des dimensions importantes au regard d'une zone colorimétrique 20. Chaque cible de localisation C1, C2 est localisée par sa forme périphérique carrée mais il va de soi que d'autres méthodes pourraient convenir.

La méthode de calibrage colorimétrique met également en oeuvre une étape d'identification E2 de chaque cible de localisation C1, C2 afin de déterminer l'orientation des cibles C1, C2 et former un référentiel robuste dans lequel la position de l'ensemble 2 des zones colorimétriques 20 est prédéterminée. Dans cet exemple, les cibles de localisation C1, C2 en « L » et en « T » sont lues et permettent de déterminer de manière précise l'orientation de chaque cible C1, C2 afin d'en déduire un référentiel brut orienté RB dans lequel se situe l'ensemble 2 des zones colorimétriques 20.

La méthode de calibrage colorimétrique met ensuite en oeuvre une étape de rectification géométrique E3 de l'image brute 80 en fonction du référentiel brut RB déterminé dans l'image brute 80. Cette étape permet de transformer le référentiel brut RB de l'image brute 80 en un référentiel standardisé RS qui peut être très aisément lu. Dans cet exemple, l'étape de rectification géométrique E3 est réalisée au moyen d'une matrice de transformation géométrique MTG. En référence à la figure 6, la matrice MTG permet la transformation géométrique du référentiel brut RB d'une cible de localisation C1 lue sur une image brute 80 en un référentiel standardisé RS de ladite cible de localisation C1 sur une image standardisée 81. La matrice de transformation géométrique MTG met en oeuvre des étapes de rotation et/ou d'homothétie sur une cible de localisation C1.

En pratique, on détermine une matrice de transformation géométrique MTG pour l'ensemble des cibles de localisation C1, C2. Autrement dit, la matrice MTG permet la transformation géométrique de l'ensemble des cibles de localisation C1 lues sur une image brute 80 en un ensemble de cibles de localisation C1, C2 standardisées.

De manière préférée, plus le nombre de cibles de localisation C1, C2 est important plus la matrice de transformation géométrique MTG est pertinente étant donné que la surface couverte les cibles de localisation est plus grande, ce qui améliore la précision. De même, plus les cibles de localisation C1, C2 sont éloignées l'une de l'autre, plus la matrice de transformation géométrique MTG est pertinente. En effet, lorsque deux cibles de localisation C1, C2 sont éloignées, celles-ci délimitent entre elles une plus grande surface.

En outre, comme l'ensemble 2 des zones colorimétriques 20 est situé entre les deux cibles de localisation C1, C2, les zones colorimétriques 20 subissent le même type de déformation que les cibles de localisation C1, C2. Aussi, la matrice de transformation géométrique MTG obtenu est pertinente pour rectifier les zones colorimétriques 20.

En référence à la figure 5, il est représenté une image rectifiée 81 dans un référentiel standardisé RS qui a été obtenu par rectification géométrique de l'image brute 80 au moyen de la matrice de transformation géométrique MTG. De manière similaire à une image brute 80, une image rectifiée 81 possède une configuration colorimétrique CC1 qui n'est pas calibrée.

Une telle image rectifiée 81 est simple et rapide à traiter étant donné que la position des zones colorimétriques 20 est parfaitement connue dans le référentiel standardisé RS défini par les cibles de localisation C1, C2. Aussi, on peut mettre en oeuvre une étape de lecture E4 des couleurs desdites zones colorimétriques 20 pour déterminer un écart colorimétrique ΔCC entre lesdites couleurs lues et lesdites couleurs étalon prédéterminées. De manière avantageuse, on obtient un écart colorimétrique ΔCC avec des ressources calculatoires limitées.

De manière avantageuse, une telle image rectifiée 81 permet de réaliser des mesures géométriques pertinentes et ainsi fournir des informations supplémentaires au dermatologue (taille de la pathologie, etc.).

Dans cet exemple, les couleurs étalon prédéterminées sont directement stockées dans une base de données en association avec une zone colorimétrique déterminée 20. Néanmoins, il va de soi que le procédé pourrait comporter une étape de lecture d'un identifiant du lot d'impression 9 et rechercher dans une base de données, pour chaque couple de données, comportant la zone colorimétrique mesurée et l'identifiant du lot d'impression 9, la couleur étalon associée.

De manière similaire à l'art antérieur, en référence à la figure 5, la méthode de calibrage met ensuite en oeuvre une étape de calibrage E5 des couleurs de ladite image brute 80 à partir dudit écart colorimétrique ΔCC déterminé afin de fournir une image calibrée 82 de configuration colorimétrique optimale CC2 au dermatologue. Autrement dit, la configuration colorimétrique CC1 de l'image brute 80 est calibrée en fonction de l'écart colorimétrique ΔCC pour obtenir la configuration colorimétrique optimale CC2.

Le dermatologue peut alors diagnostiquer l'état de l'anomalie dermatologique 7 à partir de son observation visuelle de l'image calibrée 82. De manière préférée, l'image calibrée 82 est également transmise au patient afin que celui-ci puisse l'observer.

L'utilisation de cibles de localisation C1, C2 permet d'accélérer et de rendre plus robuste la détection et la lecture des zones colorimétriques 20, ce qui est avantageux. De manière préférée, toutes les images brutes 80 du flux vidéo sont calibrées afin que le dermatologue puisse observer l'anomalie dermatologique 7 lors du déplacement de la caméra en temps réel. Une telle observation en temps réel est un avantage important.

Il a été présenté une mise en oeuvre d'un procédé de calibrage par un serveur d'intermédiation mais il va de soi que le procédé pourrait être mise en oeuvre localement sur l'ordinateur du patient ou l'ordinateur du dermatologue. En outre, le procédé de calibrage pourrait être mise en oeuvre a posteriori sur un flux vidéo enregistré.

L'invention vise également un logiciel informatique mettant en oeuvre le procédé de calibrage ainsi qu'un support informatique sur lequel est stocké ledit logiciel informatique, par exemple, un serveur informatique, un ordinateur ou un téléphone intelligent.

L'invention a été présentée pour une application dans le domaine de la dermatologie mais il va de soi qu'elle peut s'appliquer dans tout domaine de la médecine, de l'industrie (validation de couleurs de produits, etc.) et des arts (photographie, etc.).

## Revendications

1. Dispositif de calibrage colorimétrique (1) comportant un ensemble (2) de zones colorimétriques (20) de couleurs prédéterminées, dispositif **caractérisé par le fait qu'**il comporte au moins une première cible de localisation (C1) et une deuxième cible de localisation (C2) qui sont différentes de manière à former un référentiel orienté et situées au voisinage dudit ensemble (2) de zones colorimétriques (20), ledit ensemble (2) de zones colorimétriques (20) étant situé entre ladite première cible de localisation (C1) et ladite deuxième cible de localisation (C2).

2. Dispositif de calibrage (1) selon la revendication 1, dans lequel chaque cible de localisation (C1, C2) comporte au moins un motif orienté.

3. Dispositif de calibrage (1) selon l'une des revendications 1 à 2, dans lequel toutes les zones colorimétriques (20) sont situées entre ladite première cible de localisation (C1) et ladite deuxième cible de localisation (C2).

4. Dispositif de calibrage selon l'une des revendications 1 à 3, dans lequel l'ensemble (2) de zones colorimétriques (20), la première cible de localisation (C1) et la deuxième cible de localisation (C2) appartiennent à un même plan.

5. Dispositif de calibrage selon l'une des revendications 1 à 4, dans lequel le dispositif de calibrage (1) est stérile.

6. Dispositif de calibrage selon l'une des revendications 1 à 5, dans lequel chaque cible de localisation (C1, C2) est noire et blanche.

7. Dispositif de calibrage selon l'une des revendications 1 à 6, comportant :
- un support (4), sur lequel est située au moins une cible de localisation (C1, C2), comportant un logement (40) ; et
- une cartouche (5), montée de manière amovible dans le logement (40) dudit support (4), sur laquelle est situé l'ensemble (2) de zones colorimétriques (20).

8. Méthode de calibrage colorimétrique d'une image brute (80) dans laquelle est positionné un dispositif de calibrage (1) selon l'une des revendications 1 à 7, la méthode comportant :
- une étape de localisation (E1) des cibles de localisation (C1, C2) dudit dispositif de calibrage (1) dans ladite image brute (80) afin de définir un référentiel brut (RB) ;
- une étape de rectification géométrique (E3) de ladite image brute (80) à partir dudit référentiel brut (RB) afin d'obtenir une image rectifiée (81) ;
- une étape de lecture (E4) des couleurs desdites zones colorimétriques (20) dans ladite image rectifiée (81) afin de déterminer un écart colorimétrique (ΔCC) entre les couleurs lues et lesdites couleurs prédéterminées desdites zones colorimétriques (20); et
- une étape de calibrage colorimétrique (E5) de ladite image brute (80) à partir dudit écart colorimétrique (ΔCC) déterminé afin d'obtenir une image calibrée (82).

9. Procédé de calibrage colorimétrique d'un flux vidéo en temps réel par une caméra, dans lequel le procédé comporte une méthode de calibrage colorimétrique selon la revendication 8 d'une pluralité d'images dudit flux vidéo, de préférence, de chaque image dudit flux vidéo.
